Europäisches Patentamt

European Patent Office

Office européen des brevets

(11) Numéro de publication: **0 373 141**
**A2**

# (12) DEMANDE DE BREVET EUROPEEN

(21) Numéro de dépôt: **89870199.0**

(51) Int. Cl.5: **A61N 2/04**

(22) Date de dépôt: **05.12.89**

(30) Priorité: **06.12.88 BE 8801372**

(43) Date de publication de la demande:
**13.06.90 Bulletin 90/24**

(84) Etats contractants désignés:
**AT BE CH DE ES FR GB GR IT LI SE**

(71) Demandeur: **ALSTHOM INTERNATIONAL S.A.**
**Chaussée de Charleroi 148**
**B-1060 Bruxelles(BE)**

(72) Inventeur: **Frey, Guy**
**Rue de l'Elan 73**
**B-1170 Bruxelles(BE)**

(74) Mandataire: **Claeys, Pierre et al**
**Bureau Gevers rue de Livourne 7 bte 1**
**B-1050 Bruxelles(BE)**

(54) **Appareil de physiothérapie pour produire un champ magnétique à utiliser comme moyen thérapeutique.**

(57) Appareil de physiothérapie comprenant un générateur (1) de courant électrique et une bobine (3) reliée audit générateur de courant, ladite bobine comprend un solénoïde (40) et un noyau (41) fixé à l'intérieur du solénoïde et est agencée pour engendrer un champ magnétique qui est utilisé comme moyen thérapeutique. Ladite bobine a une longueur d'au moins deux fois celle du diamètre du noyau.

Fig.1.

EP 0 373 141 A2

## Appareil de physiothérapie pour produire un champ magnétique à utiliser comme moyen thérapeutique.

L'invention concerne un appareil de physiothérapie comprenant un générateur de courant électrique et une bobine reliée audit générateur de courant, ladite bobine comprend un solénoïde et un noyau fixé à l'intérieur du solénoïde et est agencée pour engendrer un champ magnétique qui est utilisé comme moyen thérapeutique.

Un tel appareil est connu et utilisé pour traiter à l'aide d'un champ magnétique une affection douloureuse. Le courant électrique fourni par le générateur est envoyé à la bobine pour créer un champ magnétique. Dans les appareils connus soit une bobine plate est introduite dans une plaquette qui est posée sur le corps à traiter, soit la bobine possède un grand diamètre et le corps à traiter est introduit dans l'enceinte formée par le solénoïde.

Pour pouvoir traiter un large éventail d'affections douloureuses il est indispensable de pouvoir créer, à l'aide de l'appareil, un champ magnétique dont les lignes de champ sont d'une bonne qualité et parviennent à atteindre, de façon précise, un endroit ou un point bien déterminé situé à l'intérieur d'un corps à traiter. Le point pouvant même se trouver relativement profondément dans le corps à traiter.

Un désavantage des appareils connus est que l'on n'obtient pas un champ magnétique dont les lignes de champ ont une qualité suffisante pour pouvoir traiter un large éventail d'affections.

L'invention a pour but de réaliser un appareil de physiothérapie produisant un champ magnétique dont les lignes de champ sont concentrées dans l'axe du noyau et dont la dispersion est réduite.

A cette fin un appareil de physiothérapie suivant l'invention est caractérisé en ce que ladite bobine a une longueur d'au moins deux fois celle du diamètre du noyau. Cette configuration de la bobine permet d'engendrer un champ magnétique ayant un haut degré d'homogénéité à l'intérieur de la bobine et permettant une concentration des lignes de champ dans l'axe du noyau, et ainsi de réduire la dispersion des lignes de champ.

Une première forme préférentielle d'un appareil suivant l'invention est caractérisée en ce que le solénoïde comporte au moins 1000 spires. Ceci permet d'engendrer un champ magnétique d'une intensité suffisante pour pouvoir traiter un grand nombre d'affections douloureuses.

Une deuxième forme préférentielle d'un appareil suivant l'invention est caractérisée en ce que le noyau est un noyau en acier dont la teneur en carbone est inférieure à 0,40 %. Ceci permet d'obtenir une bonne perméabilité magnétique relative du noyau, tout en ayant une dimension réduite du noyau.

De préférence le noyau est fabriqué en un matériau ayant une perméabilité magnétique relative qui est inférieure à 1.200. Ceci permet de limiter la grandeur du champ magnétique, et donc la dispersion des lignes de champ.

De préférence une zone située autour de l'axe central du noyau est fabriquée en un matériau ayant une perméabilité magnétique relative supérieure à celle du matériau dont est formée la zone située autour de la paroi du noyau. Ceci permet de réduire la dispersion des lignes de champ.

Une troisième forme préférentielle d'un appareil suivant l'invention est caractérisée en ce que le noyau est un noyau en acier doux étiré à froid. L'acier doux étiré à froid est particulièrement apte à être utilisé comme noyau et permet une "saturation" rapide des lignes de champ.

Une quatrième forme préférentielle d'un appareil suivant l'invention est caractérisée en ce que le générateur de courant électrique est agencé pour engendrer un courant alternatif dont l'intensité et/ou la fréquence sont variables. Ceci permet de traiter une première catégorie d'affections douloureuses.

Une cinquième forme préférentielle d'un appareil suivant l'invention est caractérisée en ce que le générateur de courant électrique est agencé pour engendrer un courant pulsé dont la fréquence et/ou l'intensité sont variables. Ceci permet de traiter une autre catégorie d'affections douloureuses.

De préférence l'appareil comporte un sélecteur de polarité. Ceci permet d'inverser la polarité du champ magnétique sans devoir modifier la position de la bobine.

De préférence la bobine est logée dans un boîtier en matière plastique pourvu d'au moins une boucle et attachée à l'aide de ladite boucle à une sangle, ladite boucle étant montée sur le boîtier de telle façon à permettre au boîtier de tourner par rapport à la boucle. La sangle permet de placer et de fixer aisément la bobine aux corps à traiter. Le fait que le boîtier peut tourner par rapport à la boucle permet d'inverser la position de la bobine et donc d'inverser la polarité du champ magnétique.

L'invention sera maintenant décrite plus en détails à l'aide d'un exemple illustré dans le dessin. Il va de soi que l'invention n'est pas limitée à l'exemple décrit et que dans le cadre de l'invention différentes variantes sont possibles. Dans le dessin :

La figure 1 montre une vue d'ensemble d'un exemple d'un appareil de physiothérapie suivant l'invention.

La figure 2 illustre à l'aide d'un schéma bloc les principaux éléments d'un générateur de courant à utiliser dans un appareil suivant l'invention.

La figure 3 montre une vue en coupe d'une bobine suivant l'invention.

La figure 4 illustre les lignes de champ d'un champ magnétique engendré à l'aide d'un appareil suivant l'invention.

La figure 5 illustre un premier type de noyau à utiliser dans une bobine suivant l'invention.

La figure 6 illustre un deuxième type de noyau à utiliser dans une bobine suivant l'invention.

La figure 7 illustre une bobine logée dans un boîtier attaché à une sangle.

Dans le dessin, les mêmes éléments ou des éléments analogues portent une même référence.

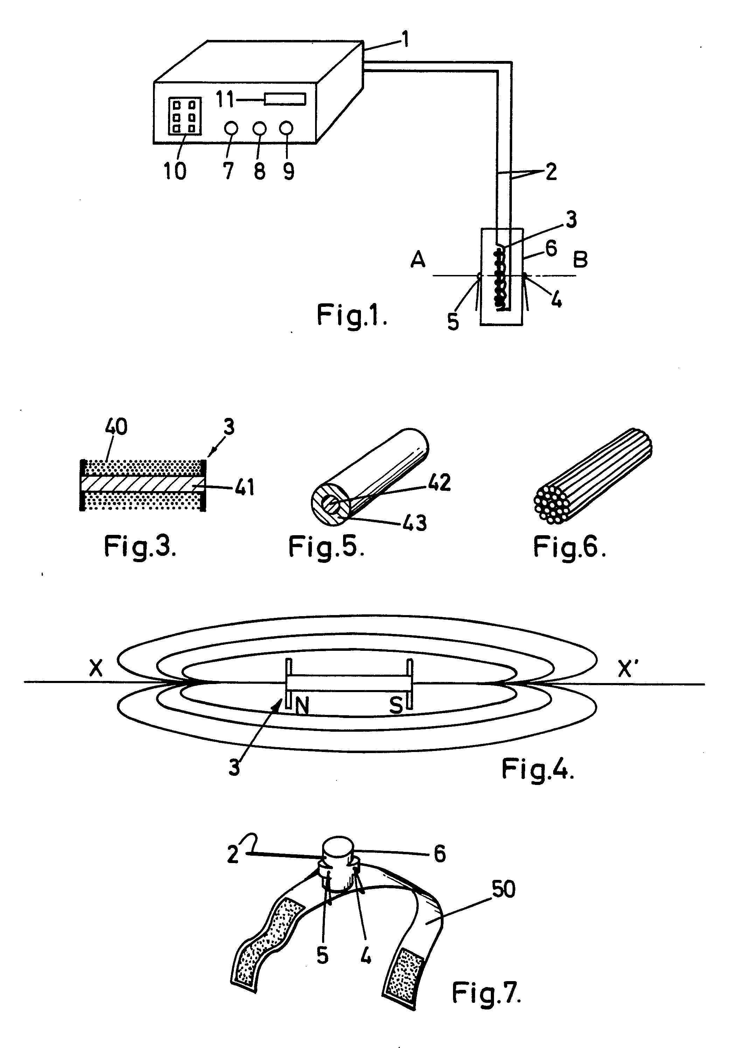

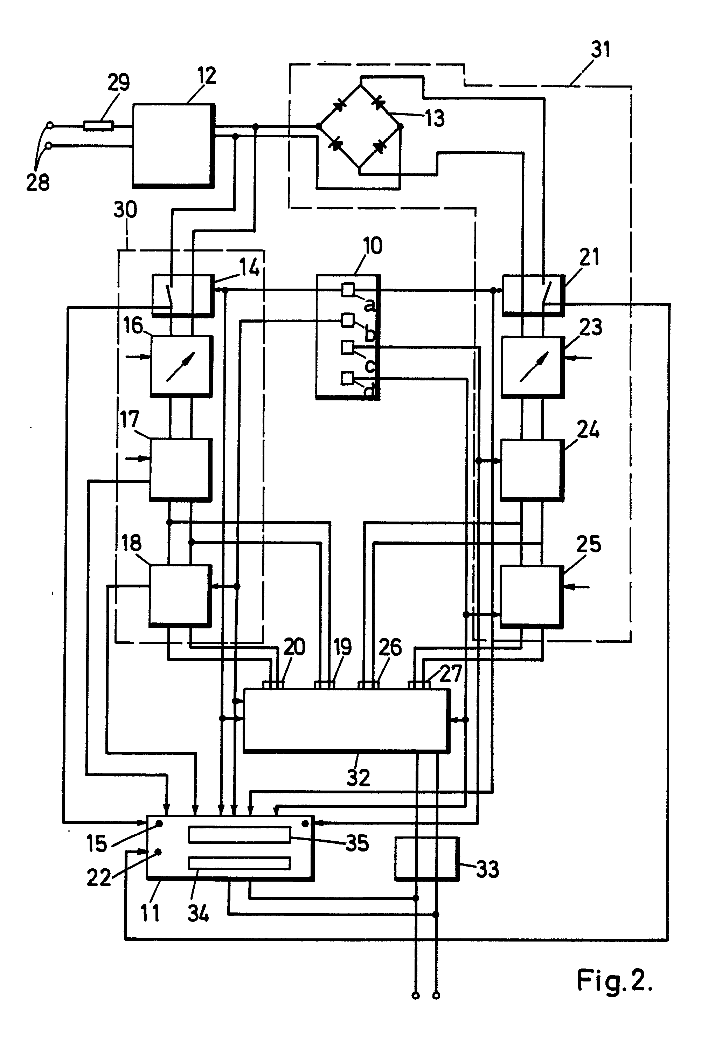

L'appareil de physiothérapie illustré à la figure 1 comporte un générateur 1 de courant électrique dont une sortie est reliée à l'aide d'un fil conducteur 2 à une bobine 3. La bobine est logée dans un boîtier 6, par exemple un boîtier en nylon ou en une autre matière plastique rigide et isolante. Le boîtier est de préférence pourvu de deux boucles 4 et 5 qui sont attachées à une sangle 50, tel qu'illustré à la figure 7, et qui permet alors de fixer le boîtier et la bobine autour d'une partie d'un corps à traiter. La sangle est de préférence pourvue de moyens adhésifs 51, 52 permettant de serrer la sangle autour de ladite partie du corps. De préférence les boucles 4 et 5 sont mobiles, permettant ainsi de faire tourner le boîtier autour de l'axe AB et donc de modifier le sens de la polarité du champ magnétique créé par la bobine lorsque cette dernière est alimentée en courant provenant du générateur 1.

Dans une autre forme de réalisation, l'appareil de physiothérapie suivant l'invention est agencé pour alimenter plusieurs bobines permettant ainsi d'appliquer un champ magnétique en plusieurs points du corps à traiter. De plus, en utilisant plusieurs bobines chacunes logées dans un boîtier et fixées à une ou plusieurs sangles tel qu'illustré à la figure 7, le fait qu'elles puissent tourner permet d'appliquer en un point un champ polarisé sud et en un autre point un champ polarisé nord.

Le générateur 1 de courant est agencé pour engendrer soit un courant continu, soit un courant alternatif, dont l'intensité est variable. Le générateur 1 est également pourvu de moyens pour faire varier la fréquence du courant. La sélection de ces différentes possibilités est réalisée à l'aide des commutateurs 7, 8 et 9 ainsi que d'un clavier de commande 10. Un tableau d'affichage 11, par exemple formé par un ensemble de LED's, permet l'affichage de la valeur du courant ainsi que d'autres informations comme par exemple la fréquence, la polarité, etc...

La figure 2 illustre à l'aide d'un schéma bloc les principaux éléments d'un générateur de courant faisant partie d'un appareil suivant l'invention. Le générateur comporte une entrée 28 à laquelle on applique une tension d'alimentation, par exemple 220 Volts en provenance d'un réseau. Cette tension est fournie à un transformateur 12 qui permet de transformer une tension primaire, par exemple de 220 Volts, en une ou plusieurs valeurs de tension secondaires, par exemple 9 Volts et/ou 12 Volts. Afin de limiter le courant fourni au générateur, un fusible 29, par exemple un fusible de 2 A, est monté dans une ligne d'arrivée du courant.

La sortie du transformateur 12 est reliée d'une part à un générateur 30 de courant alternatif et d'autre part à un générateur 31 de courant continu. Le générateur 30 de courant alternatif comporte un interrupteur 14, qui est par exemple commandé par une touche 10a du clavier de commande 10, et qui permet de brancher le générateur 30. L'interrupteur 14 est relié à un premier témoin lumineux 15, par exemple formé par un LED du tableau d'affichage 11, lequel premier témoin lumineux est allumé lorsque le générateur 30 est branché. Une sortie de l'interrupteur 14 est reliée à un régulateur de courant 16, par exemple une résistance variable. Le régulateur est positionnable (indiqué par la flèche) à l'aide d'un commutateur 7, permettant ainsi de faire varier l'intensité du courant débité. Une sortie du régulateur 16 est reliée à une entrée d'un sélecteur de fréquences 17 qui est commandé à l'aide du commutateur 8. Le sélecteur de fréquences permet de choisir la fréquence du courant alternatif fourni à la bobine et ainsi de choisir la fréquence du champ magnétique créé par la bobine 1. Le sélecteur 17 de fréquences est relié au tableau d'affichage 11, permettant ainsi d'afficher la fréquence choisie. De préférence la fréquence est variable en continu dans une plage de 1 à 100 Hz.

Une sortie du sélecteur 17 de fréquences est d'une part reliée à une première entrée 19 d'une unité de sélection 32 et d'autre part à une entrée d'un sélecteur d'alternances 18 dont une sortie est reliée à une deuxième entrée 20 de l'unité de sélection 32. Le sélecteur d'alternances 18 permet, lorsqu'il est branché, de sélectionner l'une ou l'autre alternance (demi-période) de l'onde de courant électrique et ainsi d'obtenir un champ magnétique pulsé nord ou sud, suivant que l'on ait choisi l'une ou l'autre demi-période. Le sélecteur d'alternances 18 est également relié au tableau d'affichage 11 permettant ainsi d'afficher laquelle des deux demi-périodes a été choisie. L'indication du choix d'une demi-période et le branchement de l'unité de sélection sont par exemple réalisés à l'aide d'une ou de deux touches (10b) du clavier de commande 10.

Le générateur de courant continu 31 comporte un pont de diodes 13, permettant de transformer le courant alternatif fourni à la sortie du transformateur 12 en un courant continu. Une sortie du pont de diodes 13 est reliée à un deuxième interrupteur 21, qui est par exemple également commandé par la touche 10a du clavier de commande 10, et qui permet de brancher le générateur de courant continu. La touche 10a du clavier de commande est agencée de telle sorte à empêcher que le générateur de courant continu et le générateur alternatif ne soient branchés en même temps, par exemple en utilisant un sélecteur pour la touche 10a. L'interrupteur 21 est relié à un LED 22 du tableau d'affichage qui, lorsqu'il est allumé, indique que le générateur de courant continu est branché.

Une sortie de l'interrupteur 21 est reliée à un régulateur de courant 23, par exemple une résistance variable. Le régulateur 23 est positionnable à l'aide du commutateur 7 permettant ainsi de faire varier l'intensité du courant débité. Une sortie du régulateur 23 est reliée à un dispositif inverseur 24, qui permet de choisir la polarité du courant et donc du champ magnétique. Le dispositif inverseur 24 est relié à la touche 10c du clavier de commande qui permet ainsi de positionner le dispositif inverseur 24 et donc de modifier la polarité du courant. La touche 10c est également reliée au tableau d'affichage 11 pour permettre l'affichage, par exemple à l'aide d'un signe + ou -, de la polarité choisie. Une sortie du dispositif inverseur 24 est d'une part reliée à une troisième entrée 26 de l'unité de sélection 32 et d'autre part à une entrée d'un dispositif 25 d'interruption de courant. Une entrée de commande du dispositif 25 est reliée au commutateur 9. Le dispositif 25 d'interruption est muni de moyens, par exemple formés d'un circuit RC et d'un transistor, permettant d'interrompre le courant durant des périodes de temps dont la durée peut être variable, et d'obtenir ainsi une onde rectangulaire. La manipulation du commutateur 9 permet de faire varier la durée de ladite période de temps et donc la fréquence de l'onde rectangulaire. La fréquence peut ainsi par exemple varier entre 1 et 100 Hz. Une sortie du dispositif 25 est reliée à une quatrième entrée 27 de l'unité de sélection 32. La touche 10d du clavier est reliée à une autre entrée de commande du dispositif 25 et permet de brancher le dispositif 25. La touche 10d est également reliée au tableau d'affichage 11 permettant ainsi d'afficher que le dispositif 25 est branché.

L'unité de sélection 32 comporte une première respectivement une deuxième entrée de commande reliée à la touche du clavier de commande 10a qui commande l'interrupteur 14 respectivement à la touche 10b qui commande le sélectionneur d'alternances 18. Une troisième entrée de commande de l'unité de sélection 32 est reliée à la touche 10d qui commande le dispositif 25. En fonction du choix indiqué à l'aide du clavier 10, l'unité de sélection 32 choisit un signal présent sur l'une de ses quatres entrées 19, 20, 26 et 27 et le transmet à sa sortie, qui elle est reliée par l'intermédiaire d'un limitateur de courant 33 à la bobine (non reprise dans la figure 2).

Lorsque à l'aide de la touche 10a le générateur de courant continu 31 respectivement le générateur de courant alternatif 30 a été choisi le LED 22 respectivement 15 sera allumé et l'unité de sélection 32 reliera l'une des entrées 26, 27 respectivement l'une des entrées 19, 20 avec sa sortie. Le choix entre les entrées 19, 20 respectivement les entrées 26, 27 est réalisé en fonction de la touche 10b respectivement 10c. Lorsque le sélecteur d'alternances 18 respectivement le dispositif 25 est branché, l'entrée 20 respectivement 27 sera reliée à la sortie.

La sortie du limitateur 33 de courant est également reliée au tableau d'affichage 11 permettant ainsi d'afficher sur un cadran 34 l'intensité du courant délivré à la bobine. Un cadran 35 permet d'afficher la fréquence du courant, telle que produite par le sélecteur de fréquences 17 ou le dispositif 25.

Le limitateur de courant sert, comme son nom l'indique, à limiter le courant (par exemple à maximum 200 mA) passant dans la bobine et permet ainsi de protéger la bobine.

Il va de soi que dans le cadre de l'invention diverses autres formes de réalisation pour le générateur 1 sont possibles. Ainsi dans une autre forme de réalisation le générateur 1 ne comporte pas d'unité de sélection 32 mais comporte par exemple quatre sorties individuelles auxquelles on peut chaque fois brancher la bobine. Dans ce dernier cas les touches 10a, b et d n'ont plus de raison d'être. Le générateur peut également comporter plusieurs sorties pour brancher plusieurs bobines.

Le courant électrique produit par le générateur 1 est fourni à la bobine 3 afin de créer un champ magnétique qui sera alors utilisé comme moyen thérapeutique. Cette utilisation à des fins thérapeutiques impose des normes particulières au champ magnétique engendré par la bobine, et donc à la bobine même. Ainsi les lignes de champ du champ magnétique doivent parfois être capables de pénétrer jusqu'à une distance de 40 cm dans le corps humain ou animal à traiter. De plus, les lignes de champ doivent, de préférence, être concentrées dans l'axe de la bobine et avoir une dispersion réduite. En effet, dans la magnéto-thérapie, il est important de pouvoir appliquer un champ magnétique à un point bien défini du corps qu'il faut traiter, et il faut parfois éviter que le champ magnétique soit appliqué sur des parties voisines du point qu'il faut traiter. En particulier pour des applications en accupunture cette restriction est primordiale. Il faut donc veiller à créer un champ magnétique d'une grande qualité ayant des lignes de champ d'une grande pureté. De plus, il faut aussi pour traiter certaines autres douleurs, telles par exemple des douleurs d'origine osseuse, par exemple une ostéomyélite, des champs magnétiques ayant une intensité magnétique d'au moins 7.500 Gauss. Par contre pour traiter d'autres types de douleurs plus superficielles, telles par exemple des douleurs de pathélogie tissulaire, il faut une intensité magnétique de 1.000 Gauss, ou il faut un champ pulsé comme par exemple pour traiter des douleurs inflammatoires ou des ulcères. L'intensité du champ magnétique requise pour combattre la lésion est essentiellement fonction de la profondeur à laquelle se trouve la lésion à traiter. Toutes ces différentes exigences imposent des normes très strictes à la construction de la bobine.

Afin de pouvoir créer un champ magnétique capable de traiter efficacement un large éventail de douleurs, la bobine faisant partie d'un appareil de physiothérapie suivant l'invention satisfait à des normes bien particulières qui seront décrites en détail ci-dessous.

La figure 3 montre une vue en coupe d'une bobine 3 suivant l'invention. Le solénoïde 40 comporte au moins 1000 spires, mais de préférence 2000 spires d'un fil, en particulier un fil de cuivre ayant par exemple un diamètre de 0,25 mm. Afin d'augmenter la perméabilité magnétique relative ($\mu_R$) de la bobine et donc l'intensité magnétique et le flux magnétique du champ magnétique engendré, la bobine comporte un noyau 41 fixé à l'intérieur du solénoïde. De préférence la bobine comporte une coupe circulaire.

Pour obtenir le champ magnétique répondant aux normes voulues, il est important que la bobine ait une longueur d'au moins deux fois celle du diamètre du noyau. De préférence la longueur de la bobine est à peu près quatre fois celle du diamètre du noyau. Par exemple lorsque le noyau formé par un cylindre, comporte un diamètre de 10 mm il comporte une longueur de 38 mm qui correspond également à celle de la bobine, puisque de préférence le noyau s'étend sur toute la longueur de la bobine. Ce choix de dimension de la bobine permet en effet de concentrer les lignes de champ du champ magnétique dans l'axe du noyau et donc d'engendrer un champ magnétique ayant une homogénéité considérable.

L'homogénéité du champ magnétique est encore améliorée en utilisant pour le noyau en acier dont la teneur en carbone est inférieure ou égale à 0,40 %, mais de préférence inférieure ou égale à 0,25 %. L'emploi d'un tel acier permet une plus forte concentration des lignes de champ qui de ce fait se trouvent rapidement en état de saturation dans le noyau. Ce phénomène fait apparaître un spectre de lignes de champ très dense pouvant atteindre un point situé à peu près 40 cm du centre de la bobine.

Pour obtenir un tel noyau il est nécessaire de faire subir un traitement à l'acier. Ce traitement consiste à faire subir au noyau un rapide revenu à une température de maximum 600° C, suivi d'un écrouissage très léger pendant la période de refroidissement du noyau. Durant l'opération écrouissage l'acier du noyau est soumis à un martelage permettant d'assouplir l'acier et ainsi de réduire pratiquement à zéro des courants de Foucault qui pourraient être induits dans le noyau, lorsqu'un courant électrique passe à travers le solénoïde. On obtient ainsi très peu de pertes de courants dans la bobine qui de ce fait ne chauffera que très peu durant l'emploi. De cette façon on évite également les pertes d'énergie et par conséquent cela permet de maintenir plus longtemps le champ magnétique.

Comme matériau formant le noyau il est également possible d'utiliser du fer étiré à froid (par exemple de l'acier calibré ST37), ou du fer poli. Le choix des matériaux du noyau est dicté par le fait que de préférence la perméabilité magnétique relative est inférieure à 1.200. En effet pour limiter la dispersion des lignes de champ et l'énergie transmise (une trop forte énergie pourrait causer des dégâts au corps à traiter) il est important que le champ magnétique ne dépasse pas une intensité de par exemple 10.000 Gauss à l'intérieur du corps à traiter.

La figure 4 illustre les lignes de champ d'un champ magnétique engendré à l'aide d'un appareil suivant l'invention. Comme on peut le voir sur cette figure 4, les lignes de champ sont concentrées dans l'axe XX' du noyau et n'ont que très peu de dispersion permettant ainsi de focaliser le champ magnétique sur un endroit bien déterminé du corps à traiter.

La figure 5 illustre une autre forme de réalisation d'un noyau à utiliser dans une bobine suivant l'invention. Le noyau a son centre 42 fait d'une autre qualité d'acier que celle utilisée pour faire la paroi du noyau. Par exemple la paroi est réalisée en un acier plus dur que celui utilisé pour le centre du noyau. La paroi est par exemple réalisée en acier poli alors que le centre est en acier étiré à froid. On obtient ainsi que la perméabilité magnétique relative dans une zone située autour de l'axe central du noyau est supérieure à celle de la zone située autour de la paroi du noyau.

La figure 6 illustre encore une autre forme de réalisation d'un noyau à utiliser dans une bobine suivant l'invention. Le noyau est constitué d'un assemblage de baguettes de soudage en acier cuivré, par exemple du type G-y 41 (Norme ISO 636/G233). Les ba guettes sont assemblées de telle sorte à former un cylindre ayant par exemple un diamètre de 10 mm et une longueur de 38 mm.

Le tableau ci-dessous donne, à titre d'exemple, quelques valeurs du champ magnétique engendré par l'appareil suivant l'invention. Les valeurs sont obtenues en utilisant une bobine de 38 mm de longueur avec 2000 spires et un noyau de 10 mm de diamètre fabriqué en acier doux et ayant une perméabilité magnétique relative de par exemple 500.

| Ampérage mA | Induction magnétique en Gauss mesurée au centre du noyau |
|---|---|
| 10 | 3.925 |
| 30 | 11.775 |
| 50 | 19.625 |
| 70 | 27.475 |
| 90 | 35.325 |
| 110 | 43.175 |
| 130 | 51.025 |
| 150 | 58.875 |
| 170 | 66.725 |
| 200 | 78.500 |

Grâce au diverses possibilités du générateur 1, il est possible de créer à l'aide de la bobine divers types de champs magnétiques. Ainsi en optant pour un courant continu (touche 10a) on peut créer un champ magnétique statique dont l'intensité peut par exemple varier (commutateur 7) entre 0 et 10.000 Gauss. Les champs magnétiques statiques sont utilisés lorsque le thérapeute souhaite obtenir une action antalgique ou un effet rééquilibrant du système nerveux autonome. La touche 10c permet d'inverser la polarité du courant et donc celle du champ magnétique sans pour autant devoir inverser l'orientation de la bobine. En effet lorsque le pôle nord est en contact avec le corps à traiter on obtient un effet de décontraction qui diminue le tonus musculaire, qui est vaso-dilatateur et cholinergique à prédominance anti-stress. Par contre lorsque le pôle sud est en contact avec le corps à traiter on obtient un effet de contraction qui augmente le tonus musculaire. Les champs magnétiques venant vers le pôle sud de la bobine provoquent des phénomènes de vaso- constriction qui s'opposent aux processus vaso-dilatatoires de l'inflammation.

Le dispositif d'interruption 25 permet d'obtenir un champ magnétique pulsé nord ou sud suivant la polarité indiquée par la touche 10c.

On peut également obtenir un champ magnétique pulsé à partir du générateur de courant 30 alternatif. Quelques exemples de traitement thérapeutique à l'aide de champs magnétiques pulsés sont donnés ci-dessous.

| | |
|---|---|
| - Fréquences entre 1 et 10 Hz | : Effets antalgiques |
| | Vasodilatation Augmentation ou diminution du tonus musculaire Fréquences conseillées : 2,6 Hz |
| - Fréquences entre 11 et 20 Hz | : Anti-inflammatoire |
| | Anti-oedémateux Fréquences conseillées : 12, 13, 14 Hz |
| - Fréquences entre 40 et 110 Hz | : Les fréquences comprises entre 40 et 110 Hz seront utilsées pour les actions au niveau des viscères. Les fréquences conseillées sont situées entre 42 et 55 Hz. Stimulent les leucocytes. Action sur les cellules (pompe ionique) Action cicatrisante. Traitements des ulcères. |

Enfin en branchant (touche 10b) le sélecteur d'alternance 18 on obtient un champ magnétique pulsé nord ou sud en fonction du choix de l'opérateur.

**Revendications**

1. Appareil de physiothérapie comprenant un générateur (1) de courant électrique et une bobine (3) reliée audit générateur de courant, ladite bobine comprend un solénoïde (40) et un noyau (41) fixé à l'intérieur du solénoïde et est agencée pour engendrer un champ magnétique qui est utilisé comme moyen thérapeutique, caractérisé en ce que ladite bobine a une longueur d'au moins deux fois celle du diamètre du noyau.

2. Appareil suivant la revendication 1, caractérisé en ce que le solénoïde (40) comporte au moins 1000 spires.

3. Appareil suivant la revendication 1 ou 2, caractérisé en ce que le noyau (41) est un noyau en acier dont la teneur en carbone est inférieure à 0,40 %.

4. Appareil suivant l'une des revendications 1, 2 ou 3, caractérisé en ce que le noyau (41) est fabriqué en un matériau ayant une perméabilité magnétique relative qui est inférieure à 1.200.

5. Appareil suivant la revendication 4, caractérisé en ce qu'une zone (42) située autour de l'axe central du noyau est fabriquée en un matériau ayant une perméabilité magnétique relative supérieure à celle du matériau dont est formée la zone (43) située autour de la paroi du noyau.

6. Appareil suivant l'une des revendications 1 à 4, caractérisé en ce que le noyau est un noyau en acier doux étiré à froid.

7. Appareil suivant l'une des revendications 1 à 4, caractérisé en ce que le noyau est un noyau en acier cuivré.

8. Appareil suivant l'une des revendications 1 à 4, caractérisé en ce que le noyau est formé par un ensemble de baguettes en acier cuivré.

9. Appareil suivant l'une des revendications 1 à 8, caractérisé en ce que la bobine est logée dans un boîtier (6) en matière plastique.

10. Appareil suivant l'une des revendications 1 à 9, caractérisé en ce que le générateur de courant électrique comporte un générateur (30) de courant alternatif dont l'intensité et/ou la fréquence sont variables.

11. Appareil suivant l'une des revendications 1 à 9, caractérisé en ce que le générateur de courant électrique comporte un générateur (31) de courant continu dont l'intensité est variable.

12. Appareil suivant l'une des revendications 1 à 11, caractérisé en ce que le générateur de courant électrique est agencé pour engendrer un courant pulsé (18; 25) dont la fréquence et/ou l'intensité sont variables.

13. Appareil suivant l'une des revendications 1 à 12, caractérisé en ce que le générateur de courant électrique comporte un sélecteur de polarité (24).

14. Bobine à utiliser dans un appareil suivant l'une quelconque des revendications 1 à 9 caractérisée en ce que ladite bobine a une longueur d'au moins deux fois celle du diamètre du noyau.

15. Bobine suivant la revendication 14, caractérisée en ce que le solénoïde a une longueur d'environ 32 mm et comporte à peu près 2000 spires, chaque spire ayant un diamètre d'à peu près 0,25 mm, et en ce que le noyau comporte une longueur d'environ 38 mm et un diamètre d'environ 10 mm.

16. Bobine suivant la revendication 14 ou 15, caractérisée en ce que la bobine est logée dans un boîtier en matière plastique pourvu d'au moins une boucle (5) et attaché à l'aide de ladite boucle à une sangle (50), ladite boucle étant montée sur le boîtier de telle façon à permettre au boîtier de tourner par rapport à la boucle.

17. Générateur de courant électrique à utiliser dans un appareil suivant l'une des revendications 10 à 13.

Fig.1.

Fig.3.

Fig.5.

Fig.6.

Fig.4.

Fig.7.

Fig.2.